**Europäisches Patentamt**

**European Patent Office**

**Office européen des brevets**

(11) Numéro de publication: **0 077 704**
**B1**

(12) # FASCICULE DE BREVET EUROPEEN

(45) Date de publication du fascicule du brevet:
02.05.85

(51) Int. Cl.⁴: **C 12 M 3/00**

(21) Numéro de dépôt: 82401802.2

(22) Date de dépôt: 04.10.82

(54) Procédé et dispositif de récolte de liquide allantoïque dans des oeufs embryonnés.

(30) Priorité: 05.10.81 FR 8118675

(43) Date de publication de la demande:
27.04.83 Bulletin 83/17

(45) Mention de la délivrance du brevet:
02.05.85 Bulletin 85/18

(84) Etats contractants désignés:
**DE IT NL**

(56) Documents cités:
**FR - A - 1 432 540**

**A.MAYR et al.: "Virologische Arbeitsmethoden"., Tome I, 1974, Veb Gustav Fischer Verlag, Jena (DD);**

(73) Titulaire: **INSTITUT MERIEUX (Société Anonyme), 27, rue Bourgelat B.P. 255, F-69223 Lyon Cedex 01 (FR)**

(72) Inventeur: **Galy, Michel Germain Henri, Le Biein - St Loup, F-69490 Pontcharra-sur-Turdine (FR)**

(74) Mandataire: **Lemoine, Michel et al, Cabinet Michel Lemoine 13 Boulevard des Batignolles, F-75008 Paris (FR)**

ACTORUM AG

## Description

La présente invention a trait à un procédé de récolte de liquide allantoïque chargé en substances et notamment en virus, par exemple en virus de la grippe, ou en autres substances biologiques ou chimiques, dans des œufs embryonnés et à un dispositif pour la mise en œuvre de ce procédé.

La multiplication de certains virus, et notamment du virus de la grippe, dans le but de fabriquer des vaccins viraux, s'effectue sur œufs embryonnés, notamment œufs de poules, conservés en couveuse, par exemple pendant environ 14 d, l'injection ayant lieu au onzième jour. Pour récolter le virus, on prélève le liquide allantoïque des œufs, ce liquide, chargé en virus, étant ensuite traité pour la fabrication du vaccin proprement dit.

Dans la pratique, la récolte s'effectue sur des machines, conçues à cet effet, dans lesquelles les œufs, rangés dans des alvéoles, progressent pas à pas. Les œufs passent tout d'abord dans un poste de décalottage où ils sont ouverts et inspectés visuellement, puis parviennent de là dans un poste de prélèvement. Ce dernier contient une pluralité d'aiguilles verticales au pas des œufs, ces aiguilles étant mobiles pour pénétrer dans la cavité allantoïdienne et aspirer le liquide. Les œufs sont ensuite rejetés par basculement dans une poubelle (voir par exemple le brevet FR-A N° 1432540).

L'opérateur qui inspecte visuellement les œufs décalottés sélectionne les œufs non utilisables, par exemple à l'aide d'un clavier actionnant un dispositif de commande approprié de sorte que les aiguilles correspondant à ces œufs ne pénètrent pas dans l'œuf lorsque les œufs sont arrivés au poste de prélèvement.

L'aspiration du liquide allantoïque par les aiguilles d'aspiration doit évidemment se faire sans crever le vitellus, et il est donc nécessaire de limiter suffisamment le mouvement de descente des aiguilles dans les œufs pour être certain de ne pas aspirer autre chose que le liquide allantoïque, quelle que soit la conformation interne relativement variable de l'œuf embryonnné.

L'invention a pour but de perfectionner un tel procédé et d'augmenter le rendement de la récolte sans risque de recueillir des éléments indésirables.

Un autre objectif de l'invention est de fournir un tel procédé qui se prête à une mise en œuvre automatique.

Un autre objectif encore de l'invention est de fournir un dispositif permettant de façon simple et économique la mise en œuvre de ce procédé.

L'invention a pour objet un procédé de récolte de liquide allantoïque et notamment de liquide allantoïque chargé en virus de la grippe dans des œufs embryonnés, dans lequel on décalotte les œufs et on les inspecte, puis on introduit dans la cavité allantoïdienne une aiguille ou tubulure d'aspiration pour prélever le liquide allantoïque, caractérisé par le fait que, après avoir prélevé le liquide allantoïque, on introduit dans ladite cavité un liquide aqueux en quantité suffisante pour remplir au moins partiellement ladite cavité, que l'on laisse le liquide dans la cavité pendant une durée comprise entre quelques secondes et quelques minutes, puis que l'on introduit dans la cavité une nouvelle fois une aiguille ou tubulure d'aspiration et que l'on aspire ledit liquide.

Comme liquide aqueux, on peut avantageusement utiliser une solution aqueuse dont la nature est compatible avec l'utilisation ultérieure du liquide traité pour la réalisation d'un vaccin. Ce liquide peut être avantageusement une solution de citrate de sodium, par exemple de l'ordre de 0,125M dans l'eau distillée.

En variante, on peut également utiliser de façon avantageuse une solution physiologique de chlorure de sodium ou des solutions tampons telles que par exemple le tampon P.B.S.

On peut avantageusement ajouter à ces solutions des enzymes telles que par exemple la neuraminidase ou des tensio-actifs.

En variante, on peut également utiliser une solution gazeuse formant des bulles, ladite solution pouvant être soumise, au moment de l'utilisation, à des ultrasons pour former une sorte de bouillonnement de bulles.

La durée pendant laquelle on laisse la solution dans la cavité allantoïdienne avant l'aspiration est avantageusement au moins égale à 1 min et de préférence de l'ordre de 1½ min, mais elle peut être bien plus importante selon les cas.

La quantité de liquide introduite dans la cavité allantoïdienne est de préférence de l'ordre de 7 à 8 ml par œuf.

Dans un mode de mise en œuvre préféré de l'invention, on introduit le liquide dans la cavité allantoïdienne sous la forme d'un jet à pression relativement faible. De préférence, on peut utiliser un jet conique et, de façon avantageuse, le jet peut être conçu de façon à laver la paroi de la cavité allantoïdienne au fur et à mesure de la descente de l'aiguille dans ladite cavité.

On constate, de façon surprenante, que l'invention permet d'accroître le rendement de la culture de virus sur œufs embryonnés de l'ordre de 25 à 30%, ce qui représente une grande importance pratique si l'on tient compte du très grand nombre d'œufs nécessaires à la production industrielle de vaccins.

L'invention a également pour objet un dispositif pour la mise en œuvre de ce procédé et comportant des ensembles successifs d'alvéoles recevant des œufs, des moyens pour faire progresser pas à pas lesdits ensembles d'alvéoles avec leurs œufs, un poste de décalottage, des moyens d'inspection et de sélection des œufs non utilisables, un poste de prélèvement du liquide allantoïque comprenant une pluralité d'aiguilles ou tubulures de prélèvement, à savoir une par œuf, susceptibles d'un mouvement vertical pour pénétrer dans les cavités allantoïdiennes des œufs décalottés et pour prélever par aspiration le liquide allantoïque, les aiguilles correspondant aux œufs sélectionnés non utilisables n'effectuant pas le prélèvement, et des moyens pour évacuer les œufs, caractérisé en ce qu'il comporte des moyens pour introduire dans les cavités allantoïdiennes une quantité déterminée de liquide aqueux, et des moyens comprenant des aiguilles ou tubulures d'aspiration pour prélever, après une durée d'attente, ledit liquide par aspiration.

Dans une forme de réalisation préférée, lesdits moyens d'introduction de liquide dans les cavités allantoïdiennes sont disposés en un poste situé en aval du poste de prélèvement du liquide allantoïque et, de préférence, les moyens de prélèvement dudit liquide aqueux sont également disposés en un poste situé en aval dudit poste d'introduction du liquide. Dans cette dernière forme de réalisation, la distance entre le poste d'introduction du liquide et le poste de prélèvement du liquide introduit peut avantageusement être choisie de façon telle que plusieurs ensembles d'alvéoles se déplacent simultanément entre lesdits deux postes, ce qui permet de garder le liquide introduit dans les cavités allantoïdiennes suffisamment longtemps, tout en conservant les cadences élevées de fonctionnement des dispositifs antérieurs qui ne possèdent qu'un seul poste de prélèvement.

Dans une forme de réalisation préférée du dispositif selon l'invention, les moyens d'introduction du liquide aqueux dans la cavité allantoïdienne comprennent des tubulures éventuellement susceptibles d'un ajustement vertical au-dessus des cavités allantoïdiennes, ces aiguilles se terminant par une buse formant un jet conique sous faible pression.

De façon avantageuse, l'information entrée par l'opérateur et ayant trait à la position des œufs non utilisables sélectionnés pour ne pas être récoltés, est prélevée et mémorisée pendant la durée de la progression des œufs vers le poste d'introduction de liquide, puis le poste de prélèvement de liquide introduit, de façon à ne pas actionner les buses et les aiguilles d'aspiration correspondantes.

D'autres avantages et caractéristiques de l'invention apparaîtront à la lecture de la description suivante, faite à titre d'exemple non limitatif et se référant au dessin annexé dans lequel:

la fig. 1 représente une vue schématique en perspective d'un dispositif selon l'invention;

la fig. 2 représente une vue de la partie inférieure d'une aiguille de prélèvement;

la fig. 3 représente une vue de la partie inférieure, avec une coupe partielle, d'une aiguille d'injection.

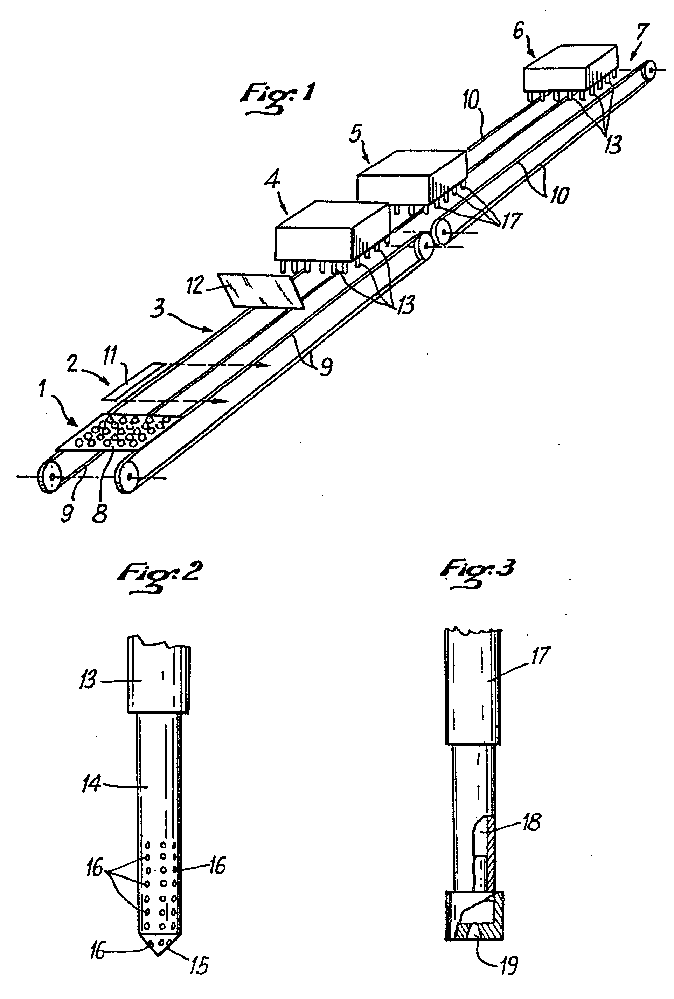

On se réfère tout d'abord à la fig. 1.

Le dispositif représenté comporte successivement les postes suivants: poste d'introduction 1, poste de décalottage 2, poste d'inspection 3, poste de prélèvement de liquide allantoïque 4, poste d'introduction de liquide 5, poste de prélèvement de liquide 6, poste d'évacuation 7 (par basculement dans une poubelle).

Les œufs sont transportés d'un poste à l'autre dans les plateaux 8 représentés de façon schématique et comportant une pluralité d'alvéoles régulièrement disposés, contenant chacun un œuf. Le transport des plateaux 8 s'effectue grâce à une première paire de chaînes latérales sans fin 9 s'étendant du poste 1 au poste 6, puis à une deuxième paire de chaînes 10 similaires, situées dans le prolongement des chaînes 9 et s'étendant du poste 5 au poste d'évacuation 7.

Bien entendu, une seule paire de chaînes de grande longueur pourrait être prévue ou tout autre moyen de convoyage.

Ces chaînes convoyeuses sont animées d'un mouvement d'avance pas à pas permettant de faire défiler les plateaux 8 successivement dans les différents postes.

Le plateau mis en place sur les chaînes au poste 1 est alors d'abord convoyé jusqu'au poste 2 où il s'immobilise. On effectue le décalottage des extrémités supérieures des œufs placés dans les alvéoles grâce, par exemple, à une lame transversalement mobile 11, les calottes sectionnées étant évacuées d'une façon en soi connue. Le plateau passe ensuite au poste 3. Un miroir 12 convenablement incliné y permet à l'opérateur de repérer ceux des œufs qui, en raison de leur état déficient, ne doivent pas faire l'objet d'un prélèvement. Il sélectionne ces œufs en introduisant, dans des moyens électroniques en soi connus, les coordonnées de chaque œuf sélectionné sur le plateau, grâce par exemple à un clavier numérique. Les informations correspondantes sont conservées dans une première mémoire pour que, lorsque le plateau 8 se trouve amené à l'étape suivante sous le poste de prélèvement 4, les œufs sélectionnés ne fassent l'objet d'aucun prélèvement.

Le poste de prélèvement 4 comporte une pluralité d'aiguilles de prélèvement 13 disposées au-dessus des chaînes de convoyage, ces aiguilles 13 étant mobiles verticalement depuis une position élevée au-dessus des œufs vers une position abaissée dans laquelle elles pénètrent dans la cavité allantoïdienne des œufs respectifs en s'arrêtant à une distance suffisante avant le fond de cette cavité pour ne pas atteindre le vitellus. Lorsque les aiguilles 13 sont mises en place dans la cavité allantoïdienne, à l'exception des aiguilles correspondant aux œufs sélectionnés qui restent maintenues dans leur position élevée, on effectue une aspiration du liquide allantoïque par les aiguilles 13. On voit sur la fig. 2 qu'une telle aiguille 13 présente avantageusement une extrémité 14 se terminant de façon conique en 15, cette extrémité étant pourvue d'une pluralité d'orifices d'aspiration 16 débouchant dans un canal central (non représenté), le liquide ainsi prélevé étant ensuite repris par des tubulures convenables vers un récipient de prélèvement. A la fin du prélèvement, qui dure par exemple 1,2 s, les aiguilles 13 se déplacent à nouveau vers le haut et sortent des cavités allandoïdiennes des œufs. Le mouvement ultérieur des chaînes 9 provoque alors le passage du plateau 8 sur les chaînes 10 et le positionnement du plateau 8 sous le poste d'introduction de liquide 5.

La quantité de liquide allantoïque recueillie au poste 4 varie en général de 6 à 13 ml par œuf et il reste en moyenne 1 ml de liquide allantoïque dans chaque œuf, cette quantité variant en fait dans des proportions assez considérables suivant les œufs individuels.

Le poste 5 comporte une pluralité d'aiguilles ou tubulures d'introduction de liquide 17, également disposées au pas des œufs dans les alvéoles et qui sont immobiles. On a représenté sur la fig. 3 une vue d'une telle aiguille 17 qui comporte un passage central 18 dans l'extrémité inférieure duquel est vissée une pièce creuse se terminant par une buse tronconique 19, de préférence avec un faible angle d'ouverture. L'injection du liquide, qui passe par le passage central 18, puis qui sort sous la forme d'un jet conique évasé hors de la buse 19, s'effectue lorsque l'œuf est situé sous l'aiguille. La distance verticale entre la partie supérieure de l'œuf décalotté et la buse 19 est de préférence réglable entre 0 et 10 mm.

Comme on le voit sur le dessin, la distance entre le poste 5 et le poste de prélèvement de liquide injecté 6 est relativement importante de façon que, entre l'injection de liquide et le prélèvement, il s'écoule une durée de 1½ min.

Le poste 6 est, en ce qui concerne sa construction, identique au poste 4 et présente une pluralité d'aiguilles 13 disposées de la même façon au pas des alvéoles. Lorsque le plateau 8 s'est immobilisé sous le poste 6, les aiguilles 13 descendent, puis pénètrent dans les cavités allantoïdiennes des œufs et aspirent le mélange formé par le liquide injecté et la quantité résiduelle de liquide allantoïque non prélevée au poste 5. Cette aspiration fournit environ 5 ml de ce mélange liquide en moyenne par œuf. Le liquide prélevé est soit traité séparément, soit de préférence purement et simplement mélangé à la récolte de liquide allantoïque provenant du poste 5.

Après relèvement des aiguilles 13 du poste 6, le plateau 8 peut être évacué par le poste d'évacuation 7.

Par ailleurs, pour chaque plateau 8, les informations concernant les œufs sélectionnés ont également été adressées à une deuxième mémoire qui permet d'éviter, pour les œufs sélectionnés, le fonctionnement des aiguilles 17 du poste 5 et des aiguilles 13 du poste 6.

Le liquide utilisé est de préférence du citrate de sodium 0,125M dans de l'eau distillée. Cependant, on peut utiliser un grand nombre d'autres solutions, telles que les solutions physiologiques de chlorure de sodium, différentes solutions salines habituellement utilisées ou toutes solutions tampons convenables.

L'augmentation de rendement en virus grippal observée en moyenne est de l'ordre de 25 à 30%, ce qui est nettement supérieur au pourcentage représenté par le volume de liquide allantoïque non récolté dans le poste 3. Ce supplément de rendement provient du fait que du virus non présent dans le liquide allantoïque est extrait par le liquide injecté, et recueilli.

Bien que l'invention ait été décrite à propos d'une forme de réalisation particulière, il est bien entendu qu'elle n'y est nullement limitée et qu'on peut lui apporter diverses modifications, sans pour cela s'éloigner ni de son cadre ni de son esprit.

**Revendications**

1. Procédé de récolte de liquide allantoïque chargé en substances et notamment en virus, par exemple en virus de la grippe, dans des œufs embryonnés, dans lequel on décalotte les œufs et on les inspecte, puis on introduit dans la cavité allantoïdienne une aiguille d'aspiration pour prélever le liquide allantoïque, caractérisé par le fait que, après avoir prélevé le liquide allantoïque, on introduit dans ladite cavité un liquide aqueux en quantité suffisante pour remplir au moins partiellement ladite cavité, que l'on laisse le liquide dans ladite cavité pendant une durée comprise entre quelques secondes et quelques minutes, puis que l'on introduit dans la cavité une nouvelle fois une aiguille d'aspiration et que l'on aspire ledit liquide.

2. Procédé selon la revendication 1, caractérisé par le fait que l'on laisse le liquide dans la cavité pendant une durée supérieure à 1 min.

3. Procédé selon l'une des revendications 1 ou 2, caractérisé en ce que l'on introduit le liquide en dirigeant un jet de liquide dans la cavité.

4. Procédé selon la revendication 3, caractérisé en ce que l'on utilise un jet conique de faible angle à faible pression.

5. Procédé selon l'une des revendications 1 à 4, caractérisé en ce qu'on utilise comme liquide un liquide choisi dans le groupe formé par les solutions de citrate de sodium, les solutions physiologiques de chlorure de sodium, les solutions tampons, et les solutions à bulles.

6. Procédé selon la revendication 5, caractérisé en ce que l'on ajoute à la solution des enzymes et/ou des tensio-actifs.

7. Procédé selon l'une des revendications 1 à 6, caractérisé en ce que la quantité de liquide injecté est de l'ordre de 7 à 8 ml/œuf.

8. Dispositif pour la mise en œuvre du procédé selon l'une des revendications 1 à 7, et comportant des ensembles successifs d'alvéoles (8) recevant des œufs, des moyens (9, 10) pour faire progresser pas à pas lesdits ensembles successifs (9) d'alvéoles recevant les œufs, un poste d'inspection et de sélection (3) des œufs non utilisables, un poste de décalottage (2), un poste de prélèvement de liquide allantoïque (4) comprenant une pluralité d'aiguilles de prélèvement (13), à savoir une par œuf, susceptibles d'un mouvement vertical pour pénétrer dans les cavités allantoïdiennes des œufs décalottés et prélever par aspiration le liquide allantoïque, les aiguilles correspondant aux œufs sélectionnés non utilisables n'effectuant pas le prélèvement, et des moyens pour évacuer les œufs, caractérisé en ce qu'il comporte des moyens (5, 17) pour introduire dans les cavités allantoïdiennes une quantité déterminée de liquide aqueux et des moyens (6) comprenant des aiguilles d'aspiration (13) pour prélever, après une durée d'attente, ledit liquide par aspiration.

9. Dispositif selon la revendication 8, caractérisé en ce que lesdits moyens d'introduction de liquide (5, 17) et lesdits moyens de prélèvement (6, 13) sont disposés en des postes (5, 6) successivement placés en aval du poste de prélèvement de liquide allantoïque (4).

10. Dispositif selon la revendication 9, caractérisé en ce qu'une distance importante sépare ledit poste d'injection (5) dudit poste de prélèvement de liquide injecté (6), afin de permettre au liquide de séjourner dans les cavités allantoïdiennes pendant la durée nécessaire.

11. Dispositif selon l'une des revendications 8 à 10, caractérisé en ce que lesdits moyens pour introduire dans les cavités allantoïdiennes une quantité déterminée de liquide aqueux comportent des aiguilles ou tubulures (17) munies d'un passage intérieur (18) débouchant dans une buse d'injection (19).

12. Dispositif selon la revendication 11, caractérisé en ce que ladite buse adresse un jet de forme généralement conique avec une faible ouverture.

## Claims

1. A process for collecting an allantoic liquid containing substances and in particular a virus, for example an influenza virus, in embryonated eggs, comprising cropping the eggs and inspecting them, then introducing in the allantoidian cavity an aspiration needle for taking off the allantoic liquid, characterized by the fact that, after having taken off the allantoic liquid, there is introduced in said cavity an aqueous liquid in sufficient quantity for at least partly filling said cavity, the liquid is left in said cavity for a period between a few seconds and a few minutes, and then an aspiration needle is again introduced in the cavity and said liquid is aspirated.

2. A process according to Claim 1, characterized by the fact that the liquid is left in the cavity for a period exceeding 1 min.

3. A process according to Claim 1 or 2, characterized in that the liquid is introduced by directing a jet of liquid in the cavity.

4. A process according to Claim 3, characterized in that a conical jet having a small included angle is used at a low pressure.

5. A process according to any of Claims 1 to 4, characterized in that there is used as liquid a liquid chosen from the group formed by solutions of sodium citrate, normal saline solutions of sodium chloride, buffer solutions, and bubble-containing solutions.

6. A process according to Claim 5, characterized in that enzymes and/or surface-active agents are added to the solution.

7. A process according to any of Claims 1 to 6, characterized in that the quantity of liquid injected is on the order of 7 to 8 ml per egg.

8. A device for carrying out the process according to any of Claims 1 to 7 and comprising successive groups of recesses (8) receiving eggs, means (9, 10) for feeding step by step said successive groups (9) of recesses receiving the eggs, an inspection and selection station (3) of the non-utilisable eggs, a cropping station (2), a station for drawing off allantoic liquid (4) comprising a plurality of drawing-off needles (13), namely one per egg, vertically movable for entering the allantoidian cavities of the cropped eggs and drawing off the allantoic liquid by aspiration, the needles corresponding to the non-utilisable selected eggs refraining from effecting the drawing-off, and means for discharging the eggs, characterized in that it comprises means (5, 17) for introducing in the allantoidian cavities a given quantity of aqueous liquid, and means (6) comprising aspiration needles (13) for drawing off, after a delayed period, said liquid by aspiration.

9. A device according to Claim 8, characterized in that said means for introducing liquid (5, 17) and said drawing-off means (6, 13) are disposed at stations (5, 6) placed in succession downstream of the station for drawing off the allantoic liquid (4).

10. A device according to Claim 9, characterized in that a great distance separates said injection station (5) from said station for drawing off the injected liquid (6), so as to allow the liquid to stay in the allantoidian cavities for the necessary period.

11. Device according to any of Claims 8 to 10, characterized in that said means for introducing in the allantoidian cavities a given quantity of aqueous liquid comprise needles or pipes (17) provided with an inner passage (18) communicating with an injection nozzle (19).

12. A device according to Claim 11, characterized in that said nozzle emits a jet having a generally conical shape with a small included angle.

## Patentansprüche

1. Verfahren zur Gewinnung von substanzenhaltiger, insbesondere virenhaltiger, z.B. grippevirenhaltiger Allantoisflüssigkeit in embryonierten Eiern, bei dem ein kalottenförmiger Schalenteil von den Eiern entfernt wird, die Eier untersucht werden und sodann eine Ansaugnadel in die allantoidische Höhlung eingeführt wird, um die Allantoisflüssigkeit zu entnehmen, dadurch gekennzeichnet, dass nach der Entnahme der Allantoisflüssigkeit eine wässerige Flüssigkeit von einer genügenden Menge in die Höhlung eingebracht wird, um die Höhlung wenigstens teilweise zu füllen, dass diese Flüssigkeit in der Höhlung während einer Zeitspanne von einigen Sekunden bis zu einigen Minuten gelassen wird, dass sodann erneut eine Ansaugnadel in die Höhlung eingeführt und die Flüssigkeit ausgesaugt wird.

2. Verfahren nach Anspruch 1, dadurch gekennzeichnet, dass die Flüssigkeit in der Höhlung während einer Zeitspanne von mehr als 1 min gelassen wird.

3. Verfahren nach einem der Ansprüche 1 oder 2, dadurch gekennzeichnet, dass die Flüssigkeit in der Weise eingebracht wird, dass ein Flüssigkeitsstrahl in die Höhlung gerichtet wird.

4. Verfahren nach Anspruch 3, dadurch gekennzeichnet, dass ein konischer Strahl mit kleinem Öffnungswinkel und mit niedrigem Druck verwendet wird.

5. Verfahren nach einem der Ansprüche 1 bis 4, dadurch gekennzeichnet, dass als Flüssigkeit eine aus der folgenden Gruppe ausgewählte Flüssigkeit verwendet wird: Natriumzitratlösungen, physiologische Kochsalzlösungen, Pufferlösungen und Lösungen mit Blasenbildung.

6. Verfahren nach Anspruch 5, dadurch gekennzeichnet, dass zu der Lösung Enzyme und/oder Netzmittel hinzugefügt werden.

7. Verfahren nach einem der Ansprüche 1 bis 6, dadurch gekennzeichnet, dass die Menge der injizierten Flüssigkeit in der Grössenordnung von 7 bis 8 ml pro Ei gewählt wird.

8. Vorrichtung zur Durchführung des Verfahrens nach einem der Ansprüche 1 bis 7, mit aufeinanderfolgenden Anordnungen (8) von Höhlen bzw. Zellen zur Eieraufnahme, mit Mitteln (9, 10) zum schrittweisen Vorrückenlassen der aufeinanderfolgenden wabenartigen Anordnungen (8) von Höhlen bzw. Zellen zur Eieraufnahme, mit einer Station (3) zum Untersuchen der Eier und zur Selektion nichtbrauchbarer Eier, mit einer Station (2) zum Entfernen kalottenförmiger Schalenteile von den Eiern, mit einer Station (4) zum Entnehmen der Allantoisflüssigkeit, wobei die Station (4) eine Anzahl von Entnahmenadeln (13), d.h. jeweils eine pro Ei, aufweist, die zu einer Vertikalbewegung befähigt sind, um in die allantoidischen Höhlungen der von kalottenförmigen Schalenteilen befreiten Eier einzudringen und durch Ansaugung die Allantoisflüssigkeit zu entnehmen, während die den als nichtbrauchbar ausgelesenen Eiern entsprechenden Nadeln keine Entnahme bewirken, und mit Mitteln zum Entleeren der Eier, gekennzeichnet durch Einrichtungen (5, 17) zum Einführen einer vor bestimmten Menge von wässeriger Flüssigkeit in die allantoidischen Höhlungen und durch eine Einrichtung (6) mit Ansaugnadeln (13) zum Entnehmen der genannten Flüssigkeit durch Ansaugen nach einer Wartezeit.

9. Vorrichtung nach Anspruch 8, dadurch gekennzeichnet, dass die Einrichtungen (5, 17) zum Einführen der Flüssigkeit und die Einrichtungen (6, 13) zum Entnehmen in Stationen (5, 6) angeordnet sind, die der Station (4) zum Entnehmen der Allantoisflüssigkeit aufeinanderfolgend nachgeschaltet sind.

10. Vorrichtung nach Anspruch 9, dadurch gekennzeichnet, dass die Station (5) zum Einführen der Flüssigkeit und die Station (6) zum Entnehmen der injizierten Flüssigkeit in einem entsprechenden Abstand voneinander angeordnet sind, um ein Verweilen der Flüssigkeit in den allantoidischen Höhlungen während der erforderlichen Zeitspanne zu erlauben.

11. Vorrichtung nach einem der Ansprüche 8 bis 10, dadurch gekennzeichnet, dass die Einrichtungen zum Einführen einer vorbestimmten Menge von wässeriger Flüssigkeit in die allantoidischen Höhlungen Nadeln oder Rohrstutzen (17) aufweisen, die jeweils mit einem inneren Durchlass (18) versehen sind, der in eine Injektionsdüse (19) mündet.

12. Vorrichtung nach Anspruch 11, dadurch gekennzeichnet, dass die Düse zum Ausstoss eines Strahls von allgemein konischer Form mit kleiner Öffnung ausgebildet ist.

Fig. 1

Fig. 2

Fig. 3